# EUROPÄISCHE PATENTSCHRIFT

(11) **EP 1 144 361 B1**
(45) Veröffentlichungstag und Bekanntmachung des Hinweises auf die Patenterteilung: **18.08.2004**
(21) Anmeldenummer: 00902598.2
(22) Anmeldetag: 15.01.2000
(51) Int. Cl.: C07C 227/40

(54) **VERFAHREN ZUR REDUKTION DES 3-DIMETHYLAMINO-2-PHENYLPROPIONSÄURE- ETHYLESTER-GEHALTS IN LÖSUNGEN VON 2-DIMETHYLAMINO -1-PHENYL -3- CYCLOHEXEN-1-CARBONSÄUREETHYLESTER**
METHOD FOR REDUCING 3-DIMETHYLAMINO-2-PHENYLPROPION-ACID ETHYL ESTER-CONTENT IN SOLUTIONS OF 2-DIMETHYLAMINO-1-PHENYL-3-CYCLOHEXENE-1-CARBOXYLIC ACID ETHYL ESTER
PROCEDE DE REDUCTION DE LA TENEUR EN ETHYLESTER D'ACIDE 3-DIMETHYLAMINO-2-PHENYLPROPIONIQUE DANS DES SOLUTIONS D'ETHYLESTER D'ACIDE 2-DIMETHYLAMINO-1-PHENYL-3-CYCLOHEXENE-1-CARBOXYLIQUE

(30) Priorität: 22.01.1999 DE 19902590
(43) Veröffentlichungstag der Anmeldung: 17.10.2001
(73) Patentinhaber: BASF AKTIENGESELLSCHAFT, 67056 Ludwigshafen (DE)
(72) Erfinder: THYES, Marco, D-67061 Ludwigshafen (DE); FALKENBERG, Wolfgang, D-67368 Westheim (DE); SCHNEIDER, Ulrich, D-66424 Homburg (DE)
(74) Vertreter: Maurer, Stefanie
(86) Internationale Anmeldenummer: PCT/EP2000/000306
(87) Internationale Veröffentlichungsnummer: WO 2000/043353

(56) Entgegenhaltungen:
- DE-B- 1 768 704
- DE-B- 2 261 462
- GB-A- 1 226 318

## Beschreibung

Die Erfindung betrifft ein Verfahren zur Reduktion des Gehalts an 3-Dimethylamino-2-phenylpropionsäureethylester (2) in 2-Dimethylamino-1-phenyl-3-cyclohexen-1-carbonsäureethylester (1), der ein Ausgangsprodukt zur Herstellung des Analgetikums Tilidin ist. Tilidin ist das trans-Isomere von 2-Dimethylamino-1-phenyl-3-cyclohexen-1-carbonsäureethylester und ist als Tilidin-Hydrochlorid-Hemihydrat im Handel.

2-Dimethylamino-1-phenyl-3-cyclohexen-1-carbonsäureethylester entsteht als cis/trans-Isomerengemisch bei der Umsetzung von Atropasäureethylester mit 1-Dimethylaminobutadien. In DE 1.923.620 wird ein Verfahren zur Herstellung von 2-Dimethylamino-1-phenyl-3-cyclohexen-1-carbonsäureethylester beschrieben, bei dem es nicht notwendig ist, das 1-Dimethylaminobutadien in isolierter Form zur Reaktion mit Atropasäureethylester einzusetzen, vielmehr verläuft das Verfahren so, daß Crotonaldehyd in Gegenwart von Kaliumcarbonat als wasserbindendem Mittel sowie katalytischen Mengen eines Chinons in einem inerten Lösungsmittel bei 3 bis 5°C mit Dimethylamin umgesetzt wird und das so erhaltene Reaktionsprodukt mit Atropasäureethylester zu 2-Dimethylamino-1-phenyl-3-cyclohexen-1-carbonsäureethylester (cis/trans-Isomerengemisch) umgesetzt wird.

Die Synthese ist begleitet von der Bildung einer Nebenkomponente, dem 3-Dimethylamino-2-phenylpropionsäureethylester.

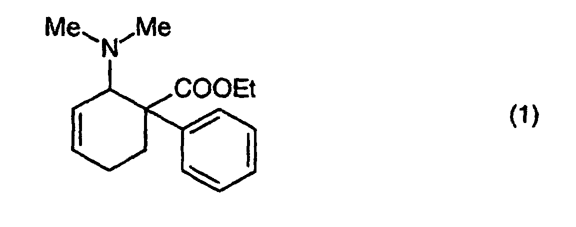

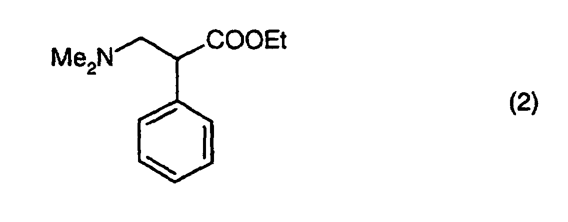

3-Dimethylamino-2-phenylpropionsäureethylester entsteht in dem Reaktionsgemisch formal durch die Addition von Dimethylamin an Atropasäureethylester, wobei Dimethylamin z.B. als Folge der Polykondensation von 1-Dimethylaminobutadien oder als Folge von Kondensationsprozessen zwischen 1-Dimethylaminobutadien und überschüssigem Crotonaldehyd freigesetzt wird.

Das Ausmaß der Bildung von 3-Dimethylamino-2-phenylpropionsäureethylester ist abhängig vom molaren Verhältnis der umgesetzten Mengen an Atropasäureethylester und Dimethylamin und wird darüber hinaus durch die Art des eingesetzten Lösungsmittels beeinflußt [Ann. Chem. 728, 64 (1969)]. Die Anwesenheit von Kaliumcarbonat während der Reaktion scheint die Bildung von 3-Dimethylamino-2-phenylpropionsäureethylester zu hemmen.

Der 3-Dimethylamino-2-phenylpropionsäureethylester wird auf dem bekannten Weg (DE 1.923.620) zur Isolierung und Reinigung des 2-Dimethylamino-1-phenyl-3-cyclohexen-1-carbonsäureethylesters (cis/trans-Isomerengemisch) nicht entfernt. Bei der zur Isolierung des Tilidins (trans-Isomeres) durchzuführenden Isomerentrennung in bekannter Weise (DE 1.923.620, GB 1.226.318), z.B. durch selektive Komplexbildung mit Zink-Ionen oder selektive Salzbildung mit Oxalsäure, findet sogar eine Anreicherung der Verunreinigung relativ zum Wirkstoff statt (DE 1.923.620), mit der Folge, daß zum Erreichen des Spezifikationswerts von maximal 0,10 % 3-Dimethylamino-2-phenylpropionsäureethylester im Tilidin-Hydrochlorid-Hemihydrat die Abtrennung z.B. durch Umkristallisation des Tilidin-Salzes vorgenommen werden muß.

Zwar wird in DE 1.923.620 angegeben, daß es bei der Herstellung von 2-Dimethylamino-1-phenyl-3-cyclohexen-1-carbonsäureethylester nach dem oben beschriebenen Verfahren prinzipiell möglich ist, einen 3-Dimethylamino-2-phenylpropionsäureethylester-Gehalt von etwa 0,1 % zu erreichen. Jedoch zeigt sich in der Praxis, daß der Gehalt bei 0,3 bis 2 % liegt.

Es bestand demnach ein Bedarf an einem einfachen, kostengünstigen Verfahren zur Reduktion des 3-Dimethylamino-2-phenylpropionsäureethylester-Gehalts in einem frühen Stadium der Tilidin-Herstellung.

Gegenstand der Erfindung ist ein Verfahren zur Reduktion des Gehalts an 3-Dimethylamino-2-phenylpropionsäureethylester in einer hiermit verunreinigten Lösung von 2-Dimethylamino-1-phenyl-3-cyclohexen-1-carbonsäureethylester in einem mit Wasser nicht mischbaren Lösungsmittel, welches darin besteht, daß man diese Lösung mit 0,75 bis 2,0 Äquivalenten einer Carbonsäure pro Mol 2-Dimethylamino-1-phenyl-3-cyclohexen-1-carbonsäureethylester versetzt und dieses Gemisch für eine Zeit im Bereich von 0,5 bis 2 Stunden bei einer Temperatur von 50°C bis 100°C rührt.

Als mit Wasser nicht mischbare Lösungsmittel eignen sich aromatische Kohlenwasserstoffe, wie Toluol, cyclische oder nicht cyclische aliphatische Kohlenwasserstoffe, wie Cyclohexan, oder aliphatische Ether, wie Diisopropylether. Als Carbonsäure eignen sich aromatische und aliphatische Carbonsäuren, wie Ameisensäure und vorzugsweise Essigsäure. Die Säure wird in einer Menge von 0,75 bis 2,0 Äquivalenten, vorzugsweise 0,75 bis 1,25 Äquivalenten, pro Mol 2-Dimethylamino-1-phenyl-3-cyclohexen-1-carbonsäureethylester eingesetzt.

Das so erhaltene Gemisch wird bei einer Temperatur von 50°C bis 100°C. vorzugsweise 70°C bis 90°C, bis zum Erreichen eines für den weiteren Herstellungsprozeß tolerierbaren Gehalts an 3-Dimethylamino-2-phenylpropionsäureethylester, in der Regel etwa 0,5 bis 2 Stunden lang, gerührt.

Nach Ende der Reaktion wird der 2-Dimethylamino-1-phenyl-3-cyclohexen-1-carbonsäureethylester in üblicher Weise aus dem Reaktionsgemisch isoliert. So kann zur Isolierung und Reinigung des Esters das Reaktionsgemisch mit Wasser versetzt und alkalisch *gemacht* werden. Anschließend kann die wäßrige Phase abgetrennt, die organische Phase gegebenenfalls mit Natriumdisulfitlösung gewaschen und eingeengt werden.

Auf diese Weise wird ein 2-Dimethylamino-1-phenyl-3-cyclohexen-1-carbon-säureethylester erhalten, der einen 3-Dimethylamino-2-phenylpropionsäureethylester-Gehalt unter 0,10 % aufweist.

Bei der Reinigung wird vorzugsweise das bei der Synthese des 2-Dimethylamino-1-phenyl-3-cyclohexen-1-carbonsäureethylesters primär anfallende cis/trans-Isomerengemisch verwendet. Eine cis/trans-Isomerisierung des 2-Dimethylamino-1-phenyl-3-cyclohexen-1-carbonsäureethylesters wird bei der Entfernung des 3-Dimethylamino-2-phenylpropionsäurethylesters nach dem neuen Verfahren praktisch nicht beobachtet. Dies steht im Gegensatz zu der beim Erhitzen des 2-Dimethylamino-1-phenyl-3-cyclohexen-1-carbonsäureethylesters in Eisessig oder wäßriger Essigsäure beobachteten Einstellung eines Isomerengleichgewichts (DE 1.951.587). Da bei dem neuen Reinigungsverfahren praktisch keine cis/trans-Isomerisierung auftritt, kann es insbesondere auch auf Tilidin selbst, also auf das trans-Isomere des 2-Dimethylamino-1-phenyl-3-cyclohexen-1-carbonsäureethylesters, angewendet werden. Selbstverständlich kann der 2-Dimethylamino-1-phenyl-3-cyclohexen-1-carbonsäureethylester für die Reinigung aber auch in der cis-Form vorliegen.

Das erfindungsgemäße Verfahren beruht formal auf der Eliminierung von Dimethylamin aus dem 3-Dimethylamino-2-phenylpropionsäureethylester. Der durch die Eliminierung gebildete Atropasäureethylester stört im weiteren Verarbeitungsprozeß nicht, kann jedoch leicht durch Extraktion der 2-Dimethylamino-1-phenyl-3-cyclohexen-1-carbonsäureethylester-Lösung mit Säure und Waschen des Säureextrakts mit einem organischen, mit Wasser nicht mischbaren Lösungsmittel entfernt werden.

Das erfindungsgemäße Verfahren bietet somit den Vorteil, bei der Tilidin-Herstellung den Gehalt an 3-Dimethylamino-2-phenylpropionsäureethylester auf einfache, schnelle und kostengünstige Weise in einem frühen Aufarbeitungsstadium so stark zu reduzieren, daß er sich im fertigen Produkt nicht mehr störend auswirkt.

### Beispiel

13,7 g (0,05 Mol) 2-Dimethylamino-1-phenyl-3-cyclohexen-1-carbonsäureethylester (cis/trans-Isomerengemisch) [Gehalt an 3-Dimethylamino-2-phenylpropionsäureethylester (HPLC): 1 %], gelöst in 40 ml Cyclohexan, wurden mit 3,0 g (0,05 Mol) Essigsäure 2 Stunden zum Rückfluß erhitzt. Nach dem Abkühlen wurden 30 ml Wasser zugesetzt. Das zweiphasige Gemisch wurde mit Natronlauge alkalisch gemacht. Anschließend wurde die Wasserphase abgetrennt. Die organische Phase wurde mit 30 ml Wasser gewaschen und eingeengt. Es wurden 13,4 g (98 %) 2-Dimethylamino-1-phenyl-3-cyclohexen-1-carbonsäureethylester-Isomerengemisch unveränderter Zusammensetzung bezüglich des cis/trans-Verhältnisses mit einem Gehalt an 3-Dimethylamino-2-phenylpropionsäureethylester von 0,05 % (HPLC) erhalten.

## Patentansprüche

1. Verfahren zur Reduktion des Gehalts an 3-Dimethylamino-2-phenylpropionsäureethylester in einer hiermit verunreinigten Lösung von 2-Dimethylamino-1-phenyl-3-cyclohexen-1-carbonsäureethylester in einem mit Wasser nicht mischbaren Lösungsmittel, **dadurch gekennzeichnet, daß** man diese Lösung mit 0,75 bis 2,0 Äquivalenten ei ner Carbonsäure pro Mol 2-Dimethylamino-1-phenyl-3-cyclohexen-1-carbonsäureethylester versetzt und dieses Gemisch für eine Zeit im Bereich von 0,5 bis 2 Stunden bei einer Temperatur von 50°C bis 100°C rührt.

## Claims

1. A process for reducing the content of ethyl 3-dimethylamino-2-phenylpropionate in a solution, contaminated therewith, of ethyl 2-dimethylamino-1-phenyl-3-cyclohexene-1-carboxylate in a water-immiscible solvent, which comprises adding from 0.75 to 2.0 equivalents of a carboxylic acid per mole of ethyl 2-dimethylamino-1-phenyl-3-cyclohexene-1-carboxylate to this solution, and stirring this mixture at a temperature of from 50°C to 100°C.

## Revendications

1. Procédé pour abaisser la teneur en 3-diméthylamino-2-phénylpropionate d'éthyle d'une solution de 2-diméthylamino-1-phényl-3-cyclohexéne-1-carboxylate d'éthyle dans un solvant non miscible à l'eau le contenant en impureté, **caractérisé par le fait que** l'on ajoute à cette solution 0,75 à 2,0 équivalents d'un acide carboxylique par mole du 2-diméthylamino-1-phényl-3-cyclohexène-1-carboxylate d'éthyle et on agite le mélange pendant une durée de 0,5 à 2 heures à une température de 50 à 100°C.
